# EUROPÄISCHE PATENTSCHRIFT

(11) **EP 1 858 574 B1**
(45) Veröffentlichungstag und Bekanntmachung des Hinweises auf die Patenterteilung: **01.09.2010**
(21) Anmeldenummer: 06706313.1
(22) Anmeldetag: 19.01.2006
(51) Int. Cl.: A61M 16/10, A61M 16/08, F16L 11/127, H05B 3/58

(54) **BEHEIZBARES SCHLAUCHSYSTEM**
HEATABLE TUBE SYSTEM
SYSTEME DE TUYAU CHAUFFABLE

(30) Priorität: 09.03.2005 DE 102005010685
(43) Veröffentlichungstag der Anmeldung: 28.11.2007
(73) Patentinhaber: Dräger Medical AG & Co. KG, 23542 Lübeck (DE)
(72) Erfinder: SCHERMEIER, Olaf, 23560 Lübeck (DE); JAGOW, Peter, 23909 Ratzeburg (DE)
(74) Vertreter: Harbsmeier, Jörn Felix
(86) Internationale Anmeldenummer: PCT/EP2006/000460
(87) Internationale Veröffentlichungsnummer: WO 2006/094576

(56) Entgegenhaltungen:
- EP-A- 0 097 901
- EP-A- 0 201 985
- WO-A-2004/011072
- US-A- 4 038 519
- US-A- 5 537 996

## Beschreibung

Die vorliegende Erfindung betrifft ein Schlauchsystem, insbesondere für ein Beatmungsgerät, mit einer Versorgungseinrichtung und mit einem Schlauch, wobei die Versorgungseinrichtung eine Spannungsversorgung mit einem ersten und einem zweiten Anschluss aufweist, wobei der Schlauch eine erste Heizleitung und eine zweite Heizleitung aufweist, wobei die erste Heizleitung mit dem ersten Anschluss und die zweite Heizleitung mit dem zweiten Anschluss lösbar verbunden ist und wobei der Schlauch eine Datenleitung zur Übertragung eines Sensorsignals aufweist, die mit der Versorgungseinrichtung lösbar verbunden ist.

Insbesondere bei medizinischen Anwendungen wie Beatmungsgeräten ist es erforderlich, dass der Schlauch, der eine Versorgungseinrichtung mit dem Patienten verbindet, beheizt ist, um beispielsweise zu verhindern, dass es zur Kondensation von Feuchtigkeit an der Wandung des Schlauches kommt und damit verbunden zu Wassertropfen, die.später in die Atemwege des Patienten gelangen können. Dazu ist aus der DE 103 12 881 B3 bekannt, die Schlauchwandung mit Heizdrähten zu versehen, um so das Innere des Schlauches aufheizen zu können.

Zur Regelung der Temperatur innerhalb des Schlauches kann es erforderlich sein, auf der Länge des Schlauches Sensoren anzubringen, um die Temperatur des Beatmungsgases messen zu können.

Aus der EP 0 201 985 A1 ist dazu bekannt, an den beiden Enden des Schlauches Temperatursensoren vorzusehen, die über eine Leitung miteinander verbunden sind. Sollen verteilt über die Länge des Schlauches mehrere, möglicherweise digitale, Temperatursensoren angeordnet werden, sind neben Heizleitungen eine Masseleitung und eine Signalleitung zum Auslesen der Sensorsignale erforderlich und damit zwei weitere Leitungen. Dies ist mit dem Nachteil vergleichsweise hoher Fertigungskosten für die Luftschläuche verbunden. Außerdem stellt sich bei der Verbindung des Schlauches mit dem Beatmungsgerät das Problem, dass wenigstens vier Kontakte erforderlich sind, wobei insbesondere die für die Datenleitungen notwendigen Kontakte hohen Qualitätsansprüchen genügen müssen.

Das Dokument US 4,038,519 offenbart ein Schlauchsystem gemäß dem Oberbegriff des Anspruchs 1.

Die US 4,038,519, von der die vorliegende Erfindung ausgeht, schlägt hierzu vor, eine der Heizleitungen wechselweise auch als eine Datenleitung zu verwenden. Dies ist jedoch mit dem Nachteil behaftet, dass eine kontinuierliche Überwachung der Temperatur innerhalb des Schlauches nicht möglich ist und daher eine Regelung der Temperatur zumindest erschwert ist.

Daher liegt der vorliegenden Erfindung ausgehend vom Stand der Technik die Aufgabe zu Grunde, ein beheizbares Schlauchsystem bereitzustellen, bei dem im Inneren des Schlauches Sensoren angebracht werden können und bei dem die Zahl der in dem Schlauch vorhandenen elektrischen Leitungen sowie die Anzahl der erforderlichen Kontakte zwischen Versorgungseinrichtung und Schlauch so gering wie möglich ist.

Diese Aufgabe wird erfindungsgemäß dadurch gelöst, dass die Versorgungseinrichtung einen Versorgungsausgang aufweist, an dem ein Versorgungspotential bereitgestellt wird, dass das Versorgungspotential zwischen dem Potential an dem ersten Anschluss und dem Potential an dem zweiten Anschluss liegt mit einer ersten Potentialdifferenz zwischen dem Versorgungsausgang und dem ersten Anschluss und einer zweiten Potentialdifferenz zwischen dem Versorgungsausgang und dem zweiten Anschluss, dass in dem Schlauch ein Sensoranschluss vorgesehen ist, der mit der ersten Heizleitung und mit der zweiten Heizleitung verbunden ist, und dass die erste Potentialdifferenz und die zweite Potentialdifferenz so gewählt sind, dass der Versorgungsausgang und der Sensoranschluss das gleiche Potential haben.

Durch die erfindungsgemäße Lösung kann bezüglich der Datenleitung in der Versorgungseinrichtung einerseits und in dem Schlauch andererseits jeweils ein Masse-Potential bereitgestellt werden, ohne dass eine zusätzliche Leitung und damit verbunden entsprechende Kontakte zwischen Versorgungseinrichtung und Schlauch erforderlich sind. Zur Bereitstellung des Masse-Potentials im Schlauch werden vielmehr die sowieso vorhandenen Heizleitungen genutzt.. Das Masse-Potential innerhalb der Versorgungseinrichtung kann an dem Versorgungsausgang abgegriffen werden, während das Masse-Potential für einen in dem Schlauch angeordneten Sensor am Sensoranschluss, der mit den beiden Heizleitungen verbunden ist, abgegriffen werden kann. Unabhängig von der Erfassung der Messsignale der in dem Schlauch angeordneten Sensoren kann die Spannungsquelle für die Heizdrähte geregelt werden, um eine gewünschte Temperatur innerhalb des Schlauches einstellen zu können.

Bevorzugt kann das gewünschte Versorgungspotential schaltungstechnisch in einfacher Weise dadurch bereitgestellt werden, dass die Spannungsversorgung als massefreie Spannungsversorgung ausgeführt ist, dass die Versorgungseinrichtung einen Versorgungsspannungsteiler aufweist, der zwischen den ersten Anschluss und den zweiten Anschluss geschaltet ist und der einen ersten Versorgungswiderstand und einen zweiten Versorgungswiderstand, die hintereinander geschaltet sind, und den dazwischen liegenden Versorgungsausgang aufweist, und dass der erste Versorgungswiderstand und der zweite Versorgungswiderstand so gewählt sind, dass der Versorgungsausgang und der Sensoranschluss das gleiche Potential haben.

Unter einer "massefreien" Spannungsversorgung im Sinne der vorliegenden Erfindung ist eine Spannungsquelle zu verstehen, die eine Potentialdifferenz zwischen zwei Anschlüssen bereitstellt, ohne dass diese Potentialdifferenz notwendig auf ein irgendwie geartetes Massepotential bezogen ist.

Weiter ist es bevorzugt, dass in dem Schlauch ein Schlauch-Spannungsteiler vorgesehen ist, der mit der ersten Heizleitung und der zweiten Heizleitung verbunden ist, dass der Schlauch-Spannungsteiler einen ersten Schlauch-Widerstand und einen zweiten Schlauch-Widerstand aufweist, die hintereinander geschaltet sind, dass der Sensoranschluss zwischen dem ersten Schlauch-Widerstand und dem zweiten Schlauch-Widerstand vorgesehen ist und dass die Schlauch-Widerstände derart gewählt sind, sodass der Versorgungsausgang und der Sensoranschluss dasselbe Potential haben.

Durch den Schlauch-Spannungsteiler kann an jeder beliebigen Stelle des Schlauches ein Sensoranschluss bereitgestellt werden, der das erforderliche Masse-Potential aufweist. Es ist lediglich erforderlich, die beiden Schlauch-Widerstände in Abhängigkeit von den Versorgungswiderständen und der Position des Anschlusses des Schlauchspannungsteilers an die Heizleitungen zu dimensionieren.

In einer dabei bevorzugten Ausführungsform ist das Verhältnis zwischen dem ersten Versorgungswiderstand und dem zweiten Versorgungswiderstand gleich dem Verhältnis zwischen dem ersten Schlauch-Widerstand und dem zweiten Schlauch-Widerstand. Hierbei handelt es sich um eine einfache Realisierung der erforderlichen Dimensionierung der Widerstände, bei der sichergestellt ist, dass der Versorgungsausgang und der Sensoranschluss dasselbe Potential aufweisen.

Dabei ist es besonders bevorzugt, wenn der erste Versorgungswiderstand und der zweite Versorgungswiderstand gleich groß sind, sodass das Verhältnis eins ist und in der Versorgungseinrichtung und in dem Schlauch jeweils die gleichen Widerstände verwendet werden können. Außerdem ist die Schaltung in der Versorgungseinrichtung und in dem Schlauch dann symmetrisch in Bezug auf die Heizleitungen. Wenn zusätzlich der erste Versorgungswiderstand und der erste Schlauch-Widerstand gleich groß sind und damit alle Widerstände den gleichen Wert aufweisen, können insgesamt identische Widerstände in dem Schlauchsystem verwendet werden.

In einer bevorzugten Ausführungsform sind die Widerstände, die der erste Heizleiter und der zweite Heizleiter zwischen ihren Enden aufweisen, gleich groß. Dies ist mit dem Vorteil verbunden, dass der Punkt, an dem die von den Anschlüssen abgewandten Enden der Heizleiter miteinander verbunden sind, das gleiche Potential wie der Versorgungsausgang aufweist, sofern der erste Versorgungswiderstand und der zweite Versorgungswiderstand gleich groß sind. Dieser Punkt kann dann ebenfalls als Sensoranschluss dienen, sodass in weiter bevorzugter Weise ein zweiter Sensor mit einem ersten Kontakt an diesem Punkt angeschlossen sein kann und mit einem zweiten Kontakt an die Datenleitung. An dem Verbindungspunkt mit den Heizleitungen wird dabei das Masse-Potential bereitgestellt.

Im Folgenden wird die vorliegende Erfindung anhand einer lediglich ein bevorzugtes Ausführungsbeispiel darstellenden Zeichnung erläutert. In der Zeichnung zeigt
- Figur 1: eine Ansicht eines Schlauchs mit einer ersten und ei- ner zweiten Heizleitung sowie mit einer Datenleitung, die in einem Anschlussstück zu einer zugehörigen Ver- sorgungseinrichtung enden, und
- Figur 2: den elektrischen Schaltplan eines erfindungsgemäßen Schlauchsystems.

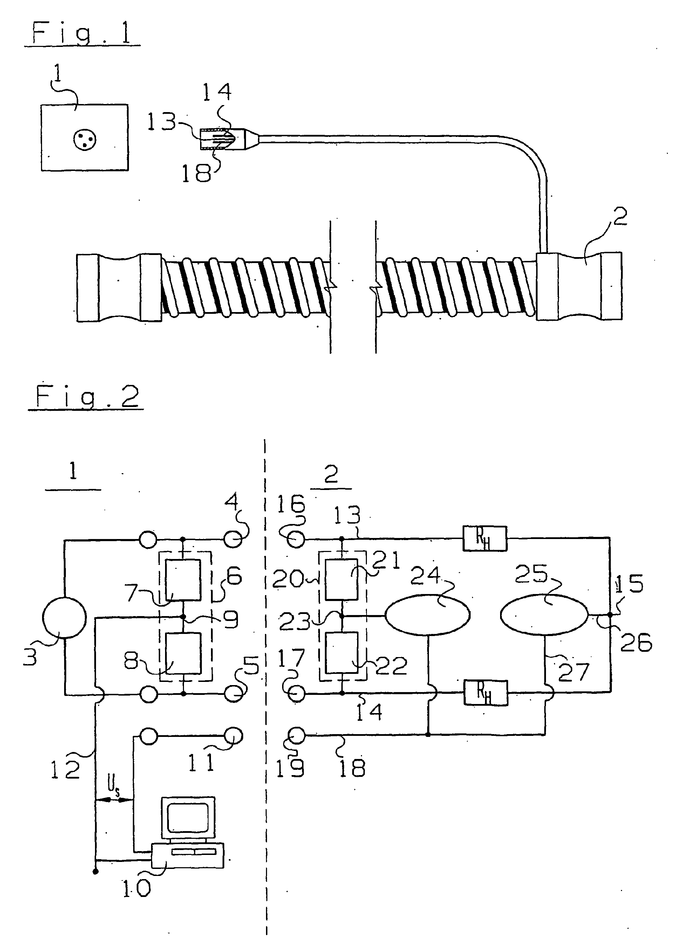

Das in Figur 2 dargestellte Ausführungsbeispiel eines erfindungsgemäßen Schlauchsystems umfasst eine Versorgungseinrichtung 1 und einen Schlauch 2, die in Figur 1 als Ansicht dargestellt sind.

Die Versorgungseinrichtung 1 ist im insoweit bevorzugten Ausführungsbeispiel mit einer massefreien Spannungsversorgung 3 versehen. Unter einer "massefreien" Spannungsversorgung 3 im Sinne dieser Anmeldung ist eine Spannungsquelle zu verstehen, die eine Potentialdifferenz bereitstellt, ohne dass diese Potentialdifferenz notwendig auf ein irgendwie geartetes Massepotential bezogen ist.

Die Spannungsversorgung 3 weist einen ersten Anschluss 4 und einen zweiten Anschluss 5 auf. Mit dem ersten Anschluss 4 und dem zweiten Anschluss 5 verbunden ist ein Versorgungsspannungsteiler 6, der einen ersten Versorgungswiderstand 7 und einen zweiten Versorgungswiderstand 8 umfasst, wobei die Versorgungswiderstände 7, 8 hintereinander geschaltet sind. Zwischen dem ersten Versorgungswiderstand 7 und dem zweiten Versorgungswiderstand 8 ist ein Versorgungsausgang 9 vorgesehen, an dem ein Versorgungspotential bereitgestellt wird, wobei das Versorgungspotential zwischen dem Potential an dem ersten Anschluss (4) und dem Potential an dem zweiten Anschluss (5) liegt.

Außerdem weist die Versorgungseinrichtung 1 eine Auswertungseinheit 10 auf, die einerseits mit einem Datenanschluss 11 verbunden ist und andererseits über eine Masseleitung 12 an den Versorgungsausgang 9 des Versorgungsspannungsteilers 6 angeschlossen ist. Zwischen dem Datenanschluss 11 und der Masseleitung 12 kann eine Signalspannung U_{S} angelegt werden, die in bevorzugter Weise 5 Volt beträgt.

Der Schlauch 2 weist eine erste Heizleitung 13 und eine zweite Heizleitung 14 auf, die im Punkt 15 miteinander verbunden sind. Außerdem weist die erste Heizleitung 13 einen ersten Heizleitungsanschluss 16 auf und die zweite Heizleitung 14 einen zweiten Heizleitungsanschluss 17. Im insoweit bevorzugten Ausführungsbeispiel weisen die Heizleitungen 13, 14 zwischen den Anschlüssen 16 bzw. 17 und dem Punkt 15 jeweils den gleichen Widerstand auf, was durch das Widerstandssymbol R_{H} angedeutet ist.

Die erste Heizleitung 13 ist über den Heizleitungsanschluss 16 lösbar mit dem ersten Anschluss 4 der Spannungsversorgung 3 verbunden. In gleicher Weise ist die zweite Heizleitung 14 über den zweiten Heizleitungsanschluss 17 mit dem zweiten Anschluss 5 der Spannungsversorgung 3 lösbar verbunden. Außerdem ist in dem Schlauch 2 eine Datenleitung 18 vorgesehen, die über einen Anschluss 19 lösbar mit dem Datenanschluss 11 der Versorgungseinrichtung 1 verbunden werden kann.

In bevorzugter Weise sind die lösbaren Verbindungen zwischen den Anschlüssen 4, 5 und 11 der Versorgungseinrichtung 1 einerseits und den Anschlüssen 16, 17 und 19 des Schlauches 2 andererseits als eine gemeinsame Steckverbindung ausgeführt, sodass der Schlauch 2 in einfacher Weise von der Versorgungseinrichtung 1 getrennt werden kann.

In diesem insoweit bevorzugten Ausführungsbeispiel weist der Schlauch 2 einen Schlauch-Spannungsteiler 20 auf, der so mit der ersten Heizleitung 13 und der zweiten Heizleitung 14 verbunden ist, dass an dem Schlauch-Spannungsteiler 20 die von der Spannungsversorgung 3 bereitgestellte Potentialdifferenz anliegt und der Einfluss des Widerstandes der Heizleitungen 13, 14 keinen Einfluss auf die an dem Schlauch-Spannungsteiler 20 anliegende Spannung hat. Der Schlauch-Spannungsteiler 20 weist einen ersten Schlauch-Widerstand 21 und einen zweiten Schlauch-Widerstand 22 auf. Zwischen dem ersten Schlauch-Widerstand 21 und dem zweiten Schlauch-Widerstand 22 und damit zwischen den Heizleitungen 13, 14 ist ein Sensoranschluss 23 vorgesehen.

Das Versorgungspotential am Versorgungsausgang 9 liegt zwischen dem Potential an dem ersten Anschluss 4 und dem Potential an dem zweiten Anschluss 5. Dabei gibt es eine erste Potentialdifferenz zwischen dem Versorgungsausgang 9 und dem ersten Anschluss 4 und eine zweite Potentialdifferenz zwischen dem Versorgungsausgang 9 und dem zweiten Anschluss 5. Die erste Potentialdifferenz und die zweite Potentialdifferenz sind erfindungsgemäß so gewählt, dass der Versorgungsausgang 9 und der Sensoranschluss 23 das gleiche Potential haben. Dies wird im insoweit bevorzugten Ausführungsbeispiel dadurch erreicht, dass die Versorgungswiderstände 7, 8, und im vorliegenden Ausführungsbeispiel auch die Schlauch-Widerstände 21, 22, so gewählt sind, dass der Versorgungsausgang 9 und der Sensoranschluss 23 unabhängig von der Spannung, die von der Spannungsversorgung 3 ausgegeben wird, das gleiche Potential aufweisen.

Dies kann weiter bevorzugt in der Weise realisiert werden, dass das Verhältnis zwischen dem ersten Versorgungswiderstand 7 und dem zweiten Versorgungswiderstand 8 gleich dem Verhältnis zwischen dem ersten Schlauch-Widerstand 21 und dem zweiten Schlauch-Widerstand 22 ist. Insbesondere können der erste Versorgungswiderstand 7 und der zweite Versorgungswiderstand 8 gleich groß sein. Sind außerdem der erste Versorgungswiderstand 7 und der erste Schlauch-Widerstand 21 gleich groß, müssen insgesamt alle Widerstände den gleichen Wert haben, um das Erfordernis des gleichen Potentials zu erfüllen. Dies ist dann mit dem Vorteil verbunden, dass vier identische Widerstände verwendet werden können.

Während im vorliegenden bevorzugten Ausführungsbeispiel der Sensoranschluss 23 durch die Verwendung des SchlauchSpannungsteilers 20 auf das gewünschte Potential gebracht wird, ist es erfindungsgemäß auch denkbar, einen Sensoranschluss vorzusehen, der mit der ersten und der zweiten Heizleitung 13, 14, vorzugsweise an deren Enden, verbunden ist und der beispielsweise allein durch die Dimensionierung der Versorgungswiderstände 7, 8 das gleiche Potential wie der Versorgungsausgang 9 aufweist.

Der Schlauch 2 weist einen ersten Temperatursensor 24 auf, der einerseits mit dem Sensoranschluss 23 und andererseits mit der Datenleitung 18 verbunden ist. Dabei dient der Sensoranschluss 23 als Masse-Anschluss für den ersten Temperatursensor 24. Die Auswertungseinheit 10 und der erste Temperatursensor 24 haben dann ein einheitliches Masse-Potential.

Schließlich ist ein zweiter Temperatursensor 25 in dem Schlauch 2 vorgesehen, der mit einem ersten Kontakt 26 mit dem Punkt 15 verbunden ist, an dem die erste Heizleitung 13 und die zweite Heizleitung 14 zusammenlaufen. Außerdem ist der zweite Temperatursensor 25 mit einem zweiten Kontakt mit der Datenleitung 18 verbunden. Dadurch, dass die erste Heizleitung 13 und die zweite Heizleitung 14 zwischen ihren Enden den gleichen Widerstand aufweisen, hat der Punkt 15 unabhängig von der Spannung, die von der Spannungsversorgung 3 bereitgestellt wird, das gleiche Potential wie der Sensoranschluss 23 und der Versorgungsausgang 9, sodass an dem Punkt 15 für den zweiten Temperatursensor 25 ebenfalls ein Masse-Potential bereitgestellt wird.

Durch die massefreie Spannungsversorgung 3 wird zunächst eine Potentialdifferenz für die Heizleitungen 13, 14 bereitgestellt. Am Versorgungsausgang 9 des Versorgungsspannungsteilers 6 kann dann ein Potential abgegriffen werden, das als Masse-Potential für die Auswertungseinheit 10 dient. Durch den Schlauch-Spannungsteiler 20 kann an dem Sensorausgang 23 ebenfalls ein Potential bereitgestellt werden, das dem am Versorgungsausgang 9 entspricht. Somit haben die Auswertungseinheit 10 und der erste Temperatursensor 24 das gleiche Masse-Potential. In analoger Weise gilt dies für den zweiten Temperatursensor 25. Dadurch können die Sensoren mit digitalem Ausgangssignal mittels eines Bus-Systems über die Datenleitung 18 ausgelesen werden, ohne dass es einer zusätzlichen Masseleitung bedarf. Das Masse-Potential wird vielmehr indirekt über die Heizleitungen 13, 14 bereitgestellt.

Dies bedeutet, dass in dem Schlauch 2 für Heizung und Sensoren 24 und 25 lediglich drei Leitungen (Heizleitungen 13, 14 und Datenleitung 18) erforderlich sind, was die Herstellungskosten des Schlauches 2 und den Aufwand für die Kontaktierung der Versorgungseinrichtung 1 reduziert.

## Patentansprüche

1. Schlauchsystem, insbesondere für ein Beatmungsgerät,
mit einer Versorgungseinrichtung (1) und
mit einem Schlauch (2),
wobei die Versorgungseinrichtung (1) eine Spannungsversorgung mit einem ersten und einem zweiten Anschluss (4, 5) aufweist,
wobei der Schlauch (2) eine erste Heizleitung (13) und eine zweite Heizleitung (14) aufweist,
wobei die erste Heizleitung (13) mit dem ersten Anschluss (4) und die zweite Heizleitung (14) mit dem zweiten Anschluss (5) lösbar verbunden ist und
wobei der Schlauch (2) eine Datenleitung (18) zur Übertragung eines Sensorsignals aufweist, die mit der Versorgungseinrichtung (1) lösbar verbunden ist,
**dadurch gekennzeichnet,**
**dass** die Versorgungseinrichtung (1) einen Versorgungsausgang (9) aufweist, an dem ein Versorgungspotential bereitgestellt wird,
**dass** das Versorgungspotential zwischen dem Potential an dem ersten Anschluss (4) und dem Potential an dem zweiten Anschluss (5) liegt mit einer ersten Potentialdifferenz zwischen dem Versorgungsausgang (9) und dem ersten Anschluss (4) und einer zweiten Potentialdifferenz zwischen dem Versorgungsausgang (9) und dem zweiten Anschluss (5), dass in dem Schlauch (2) ein Sensoranschluss (23) vorgesehen ist, der mit der ersten Heizleitung (13) und mit der zweiten Heizleitung (14) verbunden ist, und
**dass** die erste Potentialdifferenz und die zweite Potentialdifferenz so gewählt sind, dass der Versorgungsausgang (9) und der Sensoranschluss (23) das gleiche Potential haben.

2. Schlauchsystem nach Anspruch 1, **dadurch gekennzeichnet, dass** die Spannungsversorgung als massefreie Spannungsversorgung (3) ausgeführt ist,
dass die Versorgungseinrichtung (1) einen Versorgungsspannungsteiler (6) aufweist, der zwischen den ersten Anschluss (4) und den zweiten Anschluss (5) geschaltet ist und der einen ersten Versorgungswiderstand (7) und einen zweiten Versorgungswiderstand (8), die hintereinander geschaltet sind, und den dazwischen liegenden Versorgungsausgang (9) aufweist, und
dass der erste Versorgungswiderstand (7) und der zweite Versorgungswiderstand (8) so gewählt sind, dass der Versorgungsausgang (9) und der Sensoranschluss (23) das gleiche Potential haben.

3. Schlauchsystem nach Anspruch 1 oder 2, **dadurch gekennzeichnet, dass** der Schlauch (2) einen ersten Temperatursensor (24) aufweist und dass der erste Temperatursensor (24) mit dem Sensoranschluss (23) und mit der Datenleitung (18) verbunden ist.

4. Schlauchsystem nach einem der Ansprüche 1 bis 3, **dadurch gekennzeichnet, dass** in dem Schlauch (2) ein Schlauch-Spannungsteiler (20) vorgesehen ist, der mit der ersten Heizleitung (13) und der zweiten Heizleitung (14) verbunden ist, dass der Schlauch-Spannungsteiler (20) einen ersten Schlauch-Widerstand (21) und einen zweiten Schlauch-Widerstand (22) aufweist, die hintereinander geschaltet sind, dass der Sensoranschluss (23) zwischen dem ersten Schlauch-Widerstand (21) und dem zweiten Schlauch-Widerstand (22) vorgesehen ist und dass die Schlauch-Widerstände (21, 22) derart gewählt sind, dass der Versorgungsausgang (9) und der Sensoranschluss (23) dasselbe Potential haben.

5. Schlauchsystem nach Anspruch 4, **dadurch gekennzeichnet, dass** das Verhältnis zwischen dem ersten Versorgungswiderstand (7) und dem zweiten Versorgungswiderstand (8) gleich dem Verhältnis zwischen dem ersten Schlauch-Widerstand (21) und dem zweiten Schlauch-Widerstand (22) ist.

6. Schlauchsystem nach Anspruch 5, **dadurch gekennzeichnet, dass** der erste Versorgungswiderstand (7) und der zweite Versorgungswiderstand (8) gleich groß sind.

7. Schlauchsystem nach Anspruch 6, **dadurch gekennzeichnet, dass** der erste Versorgungswiderstand (7) und der erste Schlauch-Widerstand (21) gleich groß sind.

8. Schlauchsystem nach einem der Ansprüche 4 bis 7, **dadurch gekennzeichnet, dass** die Widerstände der ersten Heizleitung (13) und der zweiten Heizleitung (14) gleich groß sind.

9. Schlauchsystem nach Anspruch 8, **dadurch gekennzeichnet, dass** ein zweiter Temperatursensor (25) mit einem ersten Kontakt (26) an dem Ende des ersten Heizleiters (13) und dem Ende des zweiten Heizleiters (14) angeschlossen ist und über einen zweiten Kontakt (27) mit der Datenleitung (18) verbunden ist.

## Claims

1. Tube system, in particular for a respiration apparatus,
comprising a supply means (1) and
comprising a tube (2),
the supply means (1) having a power supply with a first and a second terminal (4, 5),
the tube (2) having a first heating line (13) and a second heating line (14),
the first heating line (13) being releasably connected to the first terminal (4) and the second heating line (14) being releasably connected to the second terminal (5), and
the tube (2) having a data line (18) for transmitting a sensor signal, which line is releasably connected to the supply means (1),
**characterised in that**
the supply means (1) has a supply output (9) at which a supply potential is provided,
the supply potential is between the potential at the first terminal (4) and the potential at the second terminal (5), with a first potential difference between the supply output (9) and the first terminal (4) and a second potential difference between the supply output (9) and the second terminal (5),
a sensor terminal (23) is provided in the tube (2) and is connected to the first heating line (13) and to the second heating line (14), and
the first potential difference and the second potential difference are selected in such a way that the supply output (9) and the sensor terminal (23) are at the same potential.

2. Tube system according to claim 1, **characterised in that**
the power supply is configured as an unearthed power supply (3),
the supply means (1) comprises a supply voltage divider (6), which is connected between the first terminal (4) and the second terminal (5) and which has a first supply resistance (7) and a second supply resistance (8), connected in series with one another, and the supply output (9) between them, and
the first supply resistance (7) and the second supply resistance (8) are selected in such a way that the supply output (9) and the sensor terminal (23) are at the same potential.

3. Tube system according to either claim 1 or claim 2, **characterised in that** the tube (2) has a first temperature sensor (24), and **in that** the first temperature sensor (24) is connected to the sensor terminal (23) and to the data line (18).

4. Tube system according to any one of claims 1 to 3, **characterised in that** a tube voltage divider (20) is provided in the tube (2) and is connected to the first heating line (13) and the second heating line (14), **in that** the tube voltage divider (20) has a first tube resistance (21) and a second tube resistance (22), connected in series with one another, **in that** the sensor terminal (23) is provided between the first tube resistance (21) and the second tube resistance (22), and **in that** the tube resistances (21, 22) are selected in such a way that the supply output (9) and the sensor terminal (23) are at the same potential.

5. Tube system according to claim 4, **characterised in that** the ratio of the first supply resistance (7) to the second supply resistance (8) is equal to the ratio of the first tube resistance (21) to the second tube resistance (22).

6. Tube system according to claim 5, **characterised in that** the first supply resistance (7) and the second supply resistance (8) are equal.

7. Tube system according to claim 6, **characterised in that** the first supply resistance (7) and the first tube resistance (21) are equal.

8. Tube system according to any one of claims 4 to 7, **characterised in that** the resistances of the first heating line (13) and the second heating line (14) are equal.

9. Tube system according to claim 8, **characterised in that** a second temperature sensor (25) is connected by a first contact (26) to the end of the first heating conductor (13) and the end of the second heating conductor (14) and via a second contact (27) to the data line (18).

## Revendications

1. Agencement de tuyau, destiné en particulier à un appareil respiratoire, comportant un dispositif d'alimentation (1) et un tuyau (2),
le dispositif d'alimentation (1) présentant une alimentation en tension électrique dotée d'une première connexion et d'une deuxième connexion (4, 5),
le tuyau (2) présentant un premier conducteur de chauffage (13) et un deuxième conducteur de chauffage (14),
le premier conducteur de chauffage (13) étant relié de façon libérable à la première connexion (4) et le deuxième conducteur de chauffage (14) étant relié de façon libérable à la deuxième connexion (5) et
le tuyau (2) présentant un conducteur (18) pour données, destiné à la transmission d'un signal de capteur, qui est relié de façon libérable au dispositif d'alimentation (1),
**caractérisé en ce que**
le dispositif d'alimentation (1) présente un départ d'alimentation (9) auquel est affecté un potentiel d'alimentation,
**en ce que** le potentiel d'alimentation se situe entre le potentiel existant à la première connexion (4) et le potentiel existant à la deuxième connexion (5), avec une première différence de potentiel entre le départ d'alimentation (9) et la première connexion (4) et une deuxième différence de potentiel entre le départ d'alimentation (9) et la deuxième connexion (5), **en ce qu'**une connexion de capteur (23) est prévue dans le tuyau (2), laquelle est reliée au premier conducteur de chauffage (13) et au deuxième conducteur de chauffage (14) et **en ce que** la première différence de potentiel et la deuxième différence de potentiel sont choisies de façon telle que le départ d'alimentation (9) et la connexion de capteur (23) présentent le même potentiel.

2. Agencement de tuyau selon la revendication 1, **caractérisé en ce que** l'alimentation en tension électrique est réalisée sous forme d'alimentation en tension électrique (3) dépourvue de masse,
**en ce que** le dispositif d'alimentation (1) présente un répartiteur (6) de tension électrique d'alimentation qui est branché entre la première connexion (4) et la deuxième connexion (5) et qui présente une première résistance d'alimentation (7) et une deuxième résistance d'alimentation (8) qui sont branchées l'une à la suite de l'autre, ainsi que le d'alimentation (9) situé entre celles-ci, et **en ce que** la première résistance d'alimentation (7) et la deuxième résistance d'alimentation (8) sont choisies de façon telle que le départ d'alimentation (9) et la connexion de capteur (23) présentent le même potentiel.

3. Agencement de tuyau selon la revendication 1 ou 2, **caractérisé en ce que** le tuyau (2) présente un premier capteur de température (24) et **en ce que** le premier capteur de température (24) est relié à la connexion de capteur (23) et au conducteur (18) pour données.

4. Agencement de tuyau selon l'une des revendications 1 à 3, **caractérisé en ce que**, dans le tuyau (2) est prévu un répartiteur de tension électrique (20) qui est relié au premier conducteur de chauffage (13) et au deuxième conducteur de chauffage (14), **en ce que** le répartiteur (20) de tension électrique de tuyau présente une première résistance de tuyau (21) et une deuxième résistance de tuyau (22) qui sont branchées l'une à la suite de l'autre, **en ce que** la connexion de capteur (23) est prévue entre la première résistance de tuyau (21) et la deuxième résistance de tuyau (22) et **en ce que** les résistances de tuyau (21, 22) sont choisies de telle manière que le départ d'alimentation (9) et la connexion de capteur (23) présentent le même potentiel.

5. Agencement de tuyau selon la revendication 4, **caractérisé en ce que** le rapport entre la première résistance d'alimentation (7) et la deuxième résistance d'alimentation (8) est égal au rapport entre la première résistance de tuyau (21) et la deuxième résistance de tuyau (22).

6. Agencement de tuyau selon la revendication 5, **caractérisé en ce que** la première résistance d'alimentation (7) et la deuxième résistance d'alimentation (8) ont la même valeur.

7. Agencement de tuyau selon la revendication 6, **caractérisé en ce que** la première résistance d'alimentation (7) et la première résistance de tuyau (21) ont la même valeur.

8. Agencement de tuyau selon l'une des revendications 4 à 7, **caractérisé en ce que** les résistances du premier conducteur de chauffage (13) et du deuxième conducteur de chauffage (14) ont la même valeur.

9. Agencement de tuyau selon la revendication 8, **caractérisé en ce qu'**un deuxième capteur de température (25) est raccordé par un premier contact (26) à l'extrémité du premier conducteur de chauffage (13) et à l'extrémité du deuxième conducteur de chauffage (14), et, par l'intermédiaire d'un deuxième contact (27), au conducteur (18) pour données.
